# EUROPEAN PATENT APPLICATION

(11) **EP 3 892 217 A1**
(43) Date of publication of application: **13.10.2021**
(21) Application number: 19892424.3
(22) Date of filing: 02.12.2019
(51) Int. Cl.: A61B 18/12

(54) **ELECTROSURGICAL GENERATOR AND ELECTROSURGICAL SYSTEM WITH VARIABLE FREQUENCY OUTPUT**

(30) Priority: 05.12.2018 CN 201811482843
(71) Applicant: Ezisurg Medical Co., Ltd., Shanghai 200000 (CN)
(72) Inventor: WENG, Jun, Shanghai 201318 (CN); GU, Shanmin, Shanghai 201318 (CN); LI, Zhidong, Shanghai 201318 (CN); ZHANG, Liping, Shanghai 201318 (CN)
(74) Representative: Mansfield, Peter Turquand
(86) International application number: PCT/CN2019/122305
(87) International publication number: WO 2020/114341

(57) **Abstract**

The present invention provides an electrosurgical generator and an electrosurgical system with variable frequency output. The electrosurgical system with variable frequency output comprises: high frequency electrosurgical instruments and an ultrasonic surgical instrument and the electrosurgical generator, wherein the high frequency electrosurgical instruments and the ultrasonic surgical instrument share one energy output interface or respectively use different energy output interfaces. An energy switching system is further arranged in the electrosurgical generator, can switch an output energy type at any time according to needs, so as to avoid frequent changes of the surgical instruments.

## Description

### Technical Field

The present invention relates to the field of electrosurgical medical instruments applicable to energy platforms for electrosurgical surgery, and in particular to an electrosurgical system capable of providing energy to a high-frequency surgical instrument and to an ultrasonic surgical instrument using the same port and at different time.

### Background Art

Currently, the field of electrosurgical medical instruments is dominated by products related to high-frequency surgical scalpels and ultrasonic scalpels, which have their own advantages and are operated based on quite different principles and implemented in quite different manners. The ultrasonic scalpels are driven and controlled mainly by acquiring higher-frequency sine wave energy by an MOS transistor operating in a linear amplification area, to drive a transducer to convert electrical energy into high-frequency mechanical vibration energy. This mode causes great loss and low efficiency. It is suitable for low-power fine operational occasions. The high-frequency electrosurgical scalpels mostly achieve outputting of high-frequency sine wave energy by performing high-frequency chopping of DC voltages using switching devices such as MOS transistor and then performing LC filtering. This mode provides high output power, high efficiency, and is widely used, but the output contains a large number of harmonic waves which has high requirements for filter design, and the high power output is likely to cause thermal damage to tissue. At present, there is a need in the market to combine a high-frequency electrosurgical scalpel with an ultrasonic scalpel, but it is difficult to integrate them with each other due to their great difference in drive control mode. If their drive control modes can be unified with each other, the circuit design will be greatly simplified and the overall performance will be improved.

At present, the commercially available solutions for the combinations of high-frequency electrosurgical scalpels and ultrasonic scalpels include a solution relating to dual electrosurgical generators proposed by Olympus and a multiplex electrosurgical generator G11 proposed by Johnson & Johnson. The solution relating to dual electrosurgical generators proposed by Olympus is based on the principle of using an ultrasonic scalpel electrosurgical generator USG-400 and a high-frequency electrosurgical scalpel electrosurgical generator ESG-400 to simultaneously supply energy to one surgical instrument. In fact, relatively great innovations are made only in the surgical instrument, and the energy supply mode is not changed much. In addition, dual electrosurgical generators are costly for users and are more complicated in use. The electrosurgical generator G11 proposed by Johnson & Johnson can be connected with either an ultrasonic scalpel or a large vessel coagulation instrument, but only one of the two instruments can be connected at a time, and the two instruments cannot be rapidly switched. Moreover, it cannot provide the functions of conventional monopolar and conventional bipolar electrosurgical scalpels, and the use of high-frequency electrosurgical scalpel instruments is restricted. In addition, G11 has lower output power and has certain limitations during use.

### Summary

In order to solve the above-mentioned existing technical problems, drive control systems for a high-frequency electrosurgical scalpel and an ultrasonic scalpel are unified in the present invention and are both operated based on a principle of pulse width modulation. Only one set of power converter and one set of drive controller are needed in an electrosurgical generator, which can output and supply high-frequency AC electrical energy to a high-frequency electrosurgical scalpel instrument, and can also output lower-frequency AC electrical energy as required by an ultrasonic scalpel instrument. The electrosurgical generator is also equipped therein with an energy switching system, which can switch the type of output energy at any time as needed, thereby avoiding frequent replacement of surgical instruments. In addition, the maximum output power of this electrosurgical generator is not less than 200 W, which can meet most market demands.

In a first aspect, the present invention provides an electrosurgical system with variable frequency output, comprising: high frequency electrosurgical instruments, an ultrasonic surgical instrument, and an electrosurgical generator, wherein the high frequency electrosurgical instruments and the ultrasonic surgical instrument share one energy output port.

Further, the electrosurgical generator can provide both high-frequency energy for an electrosurgical scalpel and low-frequency energy for an ultrasonic scalpel, but can output only one type of energy at one time, and the energy output is determined in a preemptive manner. The conversion of energy is automatically carried out by a switching network.

In another aspect, the present invention provides an electrosurgical system with variable frequency output, comprising: a combined electrosurgical and ultrasonic instrument, a connecting cable, and an electrosurgical generator, wherein the surgical instrument with combined electrosurgical and ultrasonic instrument is connected to the electrosurgical generator via the connecting cable, and the connecting cable is divided into two strands of a high-frequency cable and an ultrasonic cable, which are connected to corresponding ports on the surgical instrument with combined electrosurgical and ultrasonic instrument, respectively.

Further, the surgical instrument with combined electrosurgical and ultrasonic instrument comprises a cutting button and a coagulation button, and the surgical instrument with combined electrosurgical and ultrasonic instrument controls the electrosurgical generator to output ultrasonic energy and high-frequency electrical energy by means of the cutting button and the coagulation button, respectively.

Further, when the cutting button is pressed, the ultrasonic energy is converted into mechanical vibration energy and transmitted to a blade by an ultrasonic transducer through the cable; and when the coagulation button is pressed, the high-frequency electrical energy is transmitted to the blade and clamp forceps through the high-frequency cable, respectively.

In a further aspect, the present invention provides an electrosurgical system with variable frequency output, comprising: high frequency electrosurgical instruments, an ultrasonic surgical instrument, and an electrosurgical generator, wherein different energy output ports of the electrosurgical generator are used for the high frequency electrosurgical instruments and the ultrasonic surgical instrument, respectively.

In the above-mentioned electrosurgical system with variable frequency output according to the present invention, the electrosurgical generator also provides ports for conventional monopolar and conventional bipolar surgical instruments.

In addition, the present invention further provides an electrosurgical generator for an electrosurgical system with variable frequency output, wherein the internal system of the electrosurgical generator includes a DC (direct current) power supply system, an adjustable DC/DC converter, an inverter system, a switching system, a filter system, a control system, and an output port. The control system is used for carrying out control of energy output from the electrosurgical generator and consists of multiple modules, mainly including an integrated control system, an output energy closed-loop control module, a feedback sampling adjustable module, a switching control logic module, a DC drive module, an inverter drive module, a DC voltage and DC current sampling module, an AC (alternating current) voltage and AC current sampling module, and a switching control module.

The composition of the main power circuit of the electrosurgical generator mainly includes a DC power supply system, an adjustable DC/DC converter, an inverter system, a switching system, a filter system, and an output port. The filter system consists of two groups of filters, wherein the first group acts as a high-frequency energy output filter channel, which consists of a high-frequency isolation transformer and a low-pass filter; and the second group acts as an ultrasonic energy output filter channel, which consists of a high-frequency isolation transformer and a low-pass filter.

In the present invention, the power output principle for an ultrasonic scalpel is unified into the principle of implementation of a high-frequency electrosurgical scalpel, and the functions of both a high-frequency electrosurgical scalpel and an ultrasonic scalpel in use are achieved by using a set of electrosurgical generator, so that the design of a drive control system of the electrosurgical generator is greatly simplified, and the equipment cost is reduced. Also, frequent replacement of surgical instruments can be effectively avoided by means of a rapid energy switching system, thereby improving user experience.

### Brief Description of the Drawings

The accompanying drawings are intended to provide a further understanding of the present application, and constitute a part of the specification, and are intended to explain the present application together with specific embodiments of the present application, and do not constitute limitations on the present application.
FIG. 1 is a schematic composition diagram of an electrosurgical system with variable frequency output according to the present invention;
FIG. 2 is another schematic composition diagram of an electrosurgical system with variable frequency output according to the present invention;
FIG. 3 is another schematic composition diagram of an electrosurgical system with variable frequency output according to the present invention;
FIG. 4 is a systematic structural diagram of an electrosurgical generator of the present invention;
FIG. 5 is a first main power circuit diagram of an electrosurgical generator of the present invention;
FIG. 6 is a second main power circuit diagram of an electrosurgical generator of the present invention;
FIG. 7 is a waveform diagram of Vab and V_RF in a high-frequency energy output mode; and
FIG. 8 is a waveform diagram of Vab and V_US in an ultrasonic energy output mode.

### Detailed Description of the Embodiments

The present application will be further described below in connection with specific embodiments, wherein the drawings are only used for exemplary illustration, show only schematic diagrams, rather than physical drawings, and should not be construed as limiting the present patent. In order to better illustrate the specific embodiments of the present application, some components may be omitted, enlarged, or reduced in the drawings, and do not represent dimensions of an actual product. It will be understood by those skilled in the art that some well-known structures and descriptions thereof may be omitted in the drawings. All the other specific embodiments obtained by those of ordinary skill in the art in light of the specific embodiments of the present application without inventive efforts will fall within the scope of the present application as claimed.

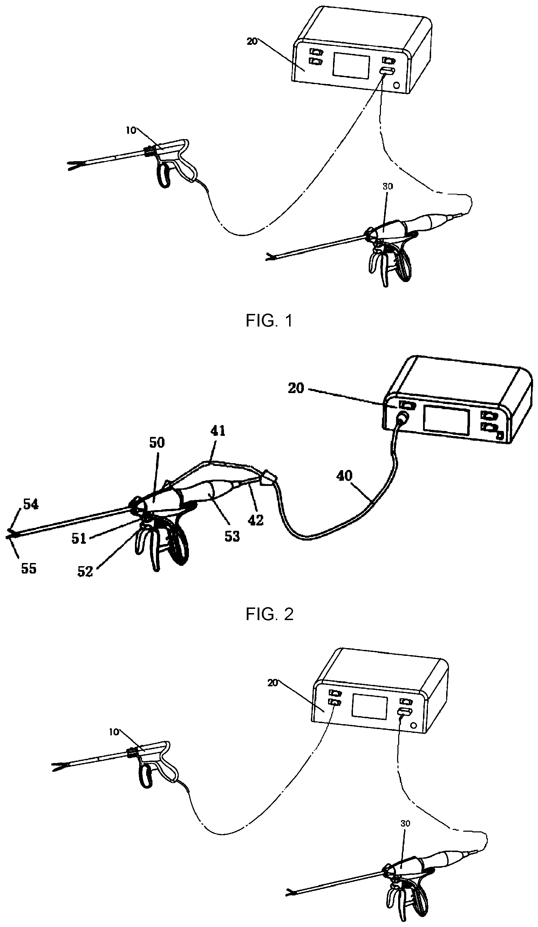

FIG. 1 is a schematic composition diagram of an electrosurgical system with variable frequency output according to the present invention. The electrosurgical system consists of high frequency electrosurgical instruments 10, an ultrasonic surgical instrument 30, and an electrosurgical generator 20, wherein the high frequency electrosurgical instruments 10 and the ultrasonic surgical instrument 30 share one energy output port 290. The electrosurgical generator 20 can provide both high-frequency energy for an electrosurgical scalpel and low-frequency energy for an ultrasonic scalpel, but can output only one type of energy at one time. The energy output is determined in a preemptive manner. In other words, energy is preferentially output to the first activated instrument and the output to the other instrument is blocked. The state is returned to the preemptive state when no instrument is activated. The conversion of the excitation energy for the instruments is automatically carried out by a switching system 240.

FIG. 2 is a schematic composition diagram of an electrosurgical system with variable frequency output according to the present invention. The electrosurgical system comprises an electrosurgical generator 20 the same as the electrosurgical generator 20 of the system of FIG. 1, and a combined electrosurgical and ultrasonic instrument 50 designed in such a manner that a high-frequency electrosurgical scalpel is integrated with an ultrasonic scalpel. The surgical instrument 50 is connected to the electrosurgical generator 20 via a connecting cable 40, and the connecting cable 40 is divided into two strands of a high-frequency cable 41 and an ultrasonic cable 42, which are connected to corresponding ports on the surgical instrument 50, respectively. The surgical instrument 50 controls the electrosurgical generator to output ultrasonic energy and high-frequency electrical energy by means of a cutting button 51 and a coagulation button 52, respectively. When the cutting button 51 is pressed, the ultrasonic energy is converted into mechanical vibration energy and transmitted to a blade 55 by an ultrasonic transducer 53 through the cable 42. When the coagulation button 52 is pressed, the high-frequency electrical energy is transmitted to the blade 55 and clamp forceps 54 through the high-frequency cable 41, respectively.

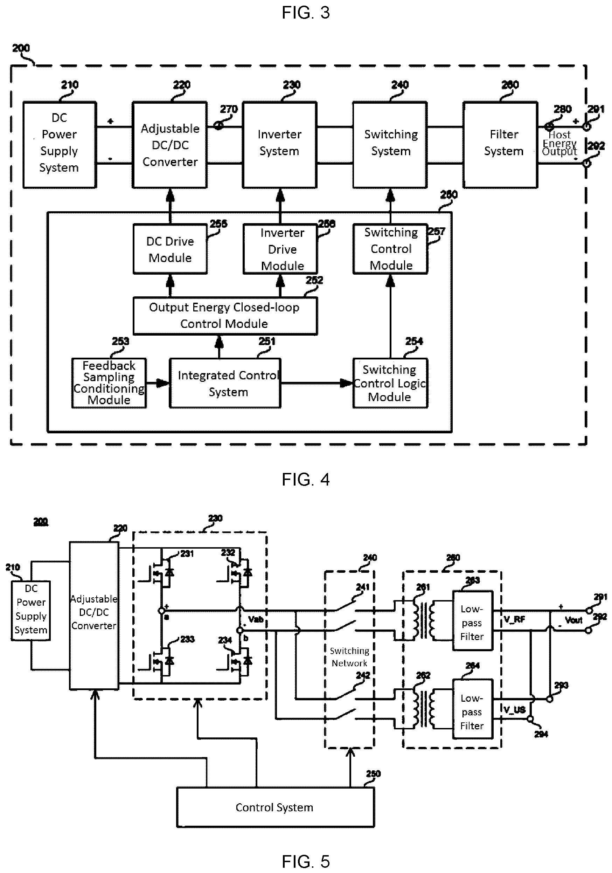

FIG. 3 is a schematic composition diagram of an electrosurgical system with variable frequency output according to the present invention. The electrosurgical system consists of high frequency electrosurgical instruments 10, an ultrasonic surgical instrument 30, and an electrosurgical generator 20, wherein different energy output ports of the electrosurgical generator are used for the high frequency electrosurgical instruments 10 and the ultrasonic surgical instrument 30, respectively. The electrosurgical generator is operated in an identical manner as that of the system 1.

As shown in FIGS. 1, 2, and 3, the electrosurgical generator provides ports for conventional monopolar and conventional bipolar surgical instruments, in addition to the ports for the surgical instrument with combined electrosurgical and ultrasonic instrument. The doctor can flexibly use them in combination as needed. However, energy can be output through only one surgical instrument port at one time.

As shown in FIG. 4, the internal system 200 of the electrosurgical generator 20 includes a DC power supply system 210, an adjustable DC/DC converter 220, an inverter system 230, a switching system 240, a filter system 260, a control system 250, and an output port 290. The control system 250 is used for carrying out control of energy output from the electrosurgical generator and consists of multiple modules, mainly including an integrated control system 251, an output energy closed-loop control module 252, a feedback sampling adjustable module 253, a switching control logic module 254, a DC drive module 255, an inverter drive module 256, a DC voltage and DC current sampling module 270, an AC voltage and AC current sampling module 280, and a switching control module 257.

As shown in FIG. 4, the feedback sampling adjustable module 253 is used for conditioning electrical quantity signals fed back from the DC voltage and DC current sampling module 270 and from the AC voltage and AC current sampling module 280 into feedback electrical quantity signals that can be used by the integrated control system 251; the integrated control system 251 is a complex control system consisting of a digital control chip such as an MCU, DSP, or FPGA, closed-loop control of output energy (by 252) can be performed according to a user instruction and the feedback electrical quantity, and at the same time a corresponding switching strategy is generated by the switching control logic module 254; the DC drive module 255 controls an output voltage from the adjustable DC/DC converter 220 according to a given output from the module 252; the inverter drive module 256 controls inversion control of the inverter system 230 according to a given output from the module 252; and the switching control system 257 controls the switching system 240 to perform a power circuit switching operation according to the switching strategy generated by the module 254.

As shown in FIG. 4, the composition of the main power circuit of the electrosurgical generator 20 mainly consists of a DC power supply system 210, an adjustable DC/DC converter 220, an inverter system 230, a switching system 240, a filter system 260, and output ports 291 and 292. The filter system 260 consists of two groups of filters, wherein the first group acts as a high-frequency energy output filter channel, which consists of a high-frequency isolation transformer 261 and a low-pass filter 263; and the second group acts as an ultrasonic energy output filter channel, which consists of a high-frequency isolation transformer 262 and a low-pass filter 264.

As shown in FIG. 5, the DC power supply system 210 supplies power to the adjustable DC/DC converter 220, and the adjustable DC/DC converter 220 controls outputting of a specified DC voltage to the inverter system 230 via the DC drive module 255 according to a given signal from the controller 250; the operation of the inverter system 230 is controlled by the control system 250 and driven by the inverter drive module 256 so that the DC voltage can be chopped into high-frequency AC square wave voltages Vab with different frequencies and different pulse widths as required, where the frequency of the AC square wave voltage Vab is linearly adjustable in a range of 300 kHz to 1 MHz. The Vab is a kind of high-frequency AC pulsed electrical energy, which must be converted into continuous AC sine wave energy by the filter system 260 before being used by a surgical instrument. The switching system 240 selects an output channel for Vab according to a given signal from the controller 250.

As shown in FIG. 5, the main circuit of the inverter system 230 may adopt any form of DC/AC converter topology, including, but not limited to, an H-bridge topology. Only the H-bridge topology is described in the present case. The H-bridge comprises four power switching devices (e.g., power MOSFETs, IGBTs, or the similar ones) 231-234, which perform switching actions according to 4-channel drive signals output from the controller, to obtain a specific PWM chopped voltage Vab.

As shown in FIG. 5, the adjustable DC/DC converter 220 can adjust a DC voltage supplied to the inverter system 230 in real time according to actual output conditions, and the output from the adjustable DC/DC converter 220 is, by default, preferentially matched with the output power from the electrosurgical generator, so that the inverter system 230 is always kept operating at a higher DC voltage utilization rate. A higher DC voltage utilization rate contributes to an increased energy conversion efficiency of the inverter system 230, and also allows for an effective reduction of the harmonic content in the output waveform. The effect is particularly significant when the system is operating in a light load state. When the AC output requires a higher crest factor, an AC voltage output with a higher crest factor is more easily obtained by cooperatively adjusting the DC voltage value, thereby obtaining a better tissue coagulation effect.

As shown in FIG. 5, the switching system 240 is used for selecting an energy output channel according to the actual operating mode. The switching system 240 switches the output channel by means of a switch. The switches may be electrically controlled physical switch (e.g., relay, contactor, or the like) or electronic switch (e.g., power switching device such as MOS, IGBT, or thyristor), and the selection of the output channel is determined by the switching strategy generated by the switching control logic module 254 in FIG. 3. During operation in a high-frequency energy output mode, the switch 241 is closed, the switch 242 is opened, and high-frequency energy is output to ports 291 and 292 via the high-frequency isolation transformer 261 and the low-pass filter 263; during operation in an ultrasonic energy output mode, the switch 241 is opened, the switch 242 is closed, and ultrasonic energy is output to ports 293 and 294 via the high-frequency isolation transformer 262 and the low-pass filter 264.

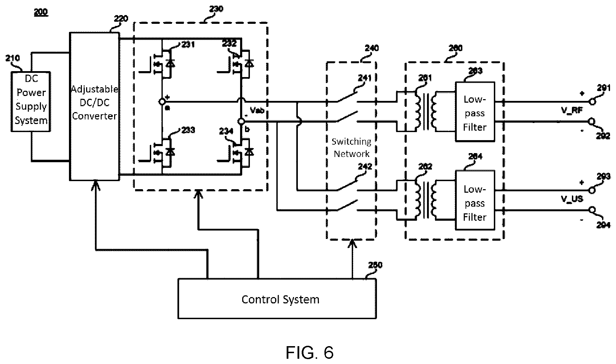

As shown in FIG. 5, the ports 291 and 292 and the ports 293 and 294 are connected in parallel, and the high-frequency energy and the ultrasonic energy are finally output through the ports 291-292. This connection mode may correspond to the first system composition scheme and the second system composition scheme. As shown in FIG. 6, the ports 291 and 292 and the ports 293 and 294 are independent of each other, the high-frequency energy is finally output through the ports 291 and 292, the ultrasonic energy is finally output through the ports 293 and 294, and they are independent of each other in terms of the output channel. This connection mode may correspond to the third system composition scheme.

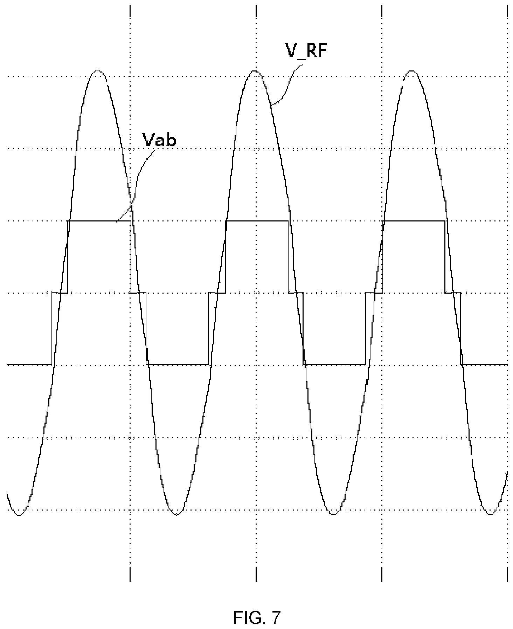

As shown in FIG. 7, Vab is a high-frequency AC pulse voltage waveform obtained when the electrosurgical generator 20 is operating in the high-frequency energy output mode, and V_RF is a high-frequency AC sine waveform obtained after passing through the high-frequency isolation boost (261) and low-pass filter (263) circuits. The Vab in the high-frequency energy output mode is a series of AC square wave voltages with a fixed phase difference and an adjustable pulse width, with an AC square wave frequency of at least 300 kHz.

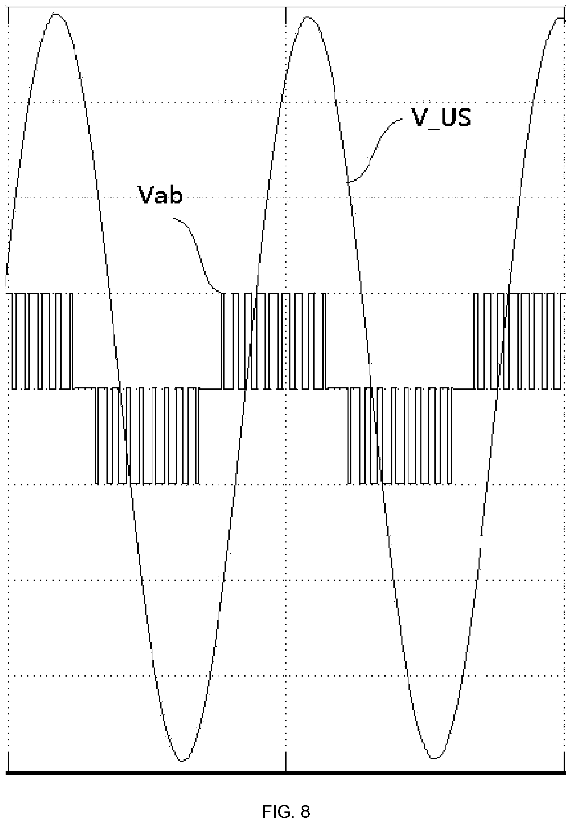

As shown in FIG. 8, Vab is a pulse width modulated voltage waveform obtained when the electrosurgical generator 20 is operating in the ultrasonic energy output mode, and V_US is an AC sine waveform obtained after passing through the high-frequency isolation boost (262) and low-pass filter (264) circuits. The Vab in the ultrasonic energy output mode is generated by means of pulse width modulation, has a fundamental frequency of generally below 100 kHz matched with a frequency required by the ultrasonic transducer, and has a carrier frequency at least 10 times higher than the fundamental frequency. After being filtered, the Vab becomes a pure sine wave voltage V_US with extremely low harmonic content, which meets the driving energy required by the ultrasonic scalpel transducer.

### Example 1

The electrosurgical system shown in FIG. 1 was used. During surgery, the doctor could use the high-frequency electrosurgical surgical instrument 10 and the ultrasonic surgical instrument 30 to output the required energy, respectively, as needed, so as to carry out the cutting, hemostasis, and coagulation of tissue.

This system does not support simultaneous activation of two instruments, but this design is reasonable, considering that undesirable consequences may be caused because a surgeon may easily be distracted when simultaneously operating two instruments. In addition, the case of simultaneous use of an ultrasonic scalpel and an electrosurgical scalpel seldom occurs in practice, therefore there is no need to be too demanding.

### Example 2

The electrosurgical system shown in FIG. 2 was used. The surgeon operated the surgical instrument with combined electrosurgical and ultrasonic instrument 50 with one hand. During surgery, the surgeon could press the cutting button 51 to carry out a tissue cutting operation with ultrasonic energy or press the coagulation button 52 to carry out an operation of coagulating tissue or a blood vessel with high-frequency energy of the electrosurgical scalpel, as needed.

This system does not support simultaneous activation of the two buttons not only in terms of the circuit, but also fool-proofing is taken into consideration in the structural design of the buttons of the instrument, to ensure that only one button can be pressed at a time. This is because the cutting and coagulation operations cannot be performed simultaneously in an actual surgical process.

### Example 3

The electrosurgical system shown in FIG. 3 was used. During surgery, the surgeon could use the high-frequency electrosurgical surgical instrument 10 and the ultrasonic surgical instrument 30 to output the required energy, respectively, as needed, so as to carry out the cutting, hemostasis, and coagulation of tissue.

This system does not support simultaneous activation of two instruments, but this design is reasonable, considering that undesirable consequences may be caused because a surgeon may easily be distracted when simultaneously operating two instruments. In addition, the case of simultaneous use of an ultrasonic scalpel and an electrosurgical scalpel seldom occurs in practice, therefore there is no need to be too demanding.

It should be noted that the embodiments in the drawings are merely representative examples of the present invention. It will be readily understood by those skilled in the art that the scope of protection of the present invention is not limited to the scope defined by the embodiments in the drawings, and combinations, variations, and changes of the embodiments in the drawings shall fall within the scope of protection of the present invention.

Only the preferred embodiments of the present invention are disclosed above, and the scope of the present invention as claimed is, of course, not limited thereto. Therefore, equivalent changes made based on the claims of the present invention shall still fall within the scope covered by the present invention.

## Claims

1. An electrosurgical system with variable frequency output, comprising: high frequency electrosurgical instruments (10), an ultrasonic surgical instrument (30) and an electrosurgical generator (20), **characterized in that** the high frequency electrosurgical instruments (10) and the ultrasonic surgical instrument (30) share one energy output port (290).

2. The electrosurgical system with variable frequency output according to claim 1, **characterized in that** the electrosurgical generator (20) is able to provide both high-frequency energy for an electrosurgical scalpel and low-frequency energy for an ultrasonic scalpel, but is able to output only one type of energy at one time, and an energy output is determined in a preemptive manner.

3. The electrosurgical system with variable frequency output according to claim 1, **characterized in that** a conversion of energy is automatically carried out by a switching network (240).

4. The electrosurgical system with variable frequency output according to claim 1, **characterized in that** the electrosurgical generator (20) further provides ports for conventional monopolar and conventional bipolar surgical instruments.

5. An electrosurgical system with variable frequency output, comprising: a combined electrosurgical and ultrasonic instrument (50), a connecting cable (40) and an electrosurgical generator (20), **characterized in that** the surgical instrument with combined electrosurgical and ultrasonic instrument (50) is connected to the electrosurgical generator (20) via the connecting cable (40), and the connecting cable (40) is divided into two strands of a high-frequency cable (41) and an ultrasonic cable (42), which are connected to corresponding ports on the surgical instrument with combined electrosurgical and ultrasonic instrument (50), respectively.

6. The electrosurgical system with variable frequency output according to claim 5, **characterized in that** the surgical instrument with combined electrosurgical and ultrasonic instrument (50) comprises a cutting button (51) and a coagulation button (52), wherein the surgical instrument (50) controls the electrosurgical generator (20) to respectively output ultrasonic energy and high-frequency electrical energy by means of the cutting button (51) and the coagulation button (52).

7. The electrosurgical system with variable frequency output according to claim 6, **characterized in that** when the cutting button (51) is pressed, the ultrasonic energy is converted into mechanical vibration energy and transmitted to a blade (55) by an ultrasonic transducer (53) through the cable (42); and when the coagulation button (52) is pressed, the high-frequency electrical energy is transmitted to the blade (55) and a clamp forceps (54) through the high-frequency cable (41), respectively.

8. The electrosurgical system with variable frequency output according to claim 5, **characterized in that** the electrosurgical generator (20) further provides ports for conventional monopolar and conventional bipolar surgical instruments.

9. An electrosurgical system with variable frequency output, comprising: high frequency electrosurgical instruments (10), an ultrasonic surgical instrument (30) and an electrosurgical generator (20), **characterized in that** different energy output ports of the electrosurgical generator are used for the high frequency electrosurgical instruments (10) and the ultrasonic surgical instrument (30), respectively.

10. The electrosurgical system with variable frequency output according to claim 9, **characterized in that** the electrosurgical generator (20) further provides ports for conventional monopolar and conventional bipolar surgical instruments.

11. An electrosurgical generator (20) for the electrosurgical system with variable frequency output according to any one of claims 1 to 10, **characterized in that** the electrosurgical generator (20) comprises an internal system (200), **characterized in that** the internal system (200) comprises a DC power supply system (210), an adjustable DC/DC converter (220), an inverter system (230), a switching system (240), a filter system (260), a control system (250) and an output port (290), wherein the control system (250) is configured to carry out control of energy output from the electrosurgical generator (20).

12. The electrosurgical generator (20) according to claim 11, **characterized in that** the internal system (200) further comprises a DC voltage and DC current sampling module (270), an AC voltage and AC current sampling module (280) and a switching control module (257).

13. The electrosurgical generator (20) according to claim 12, **characterized in that** the control system (250) mainly comprises an integrated control system (251), an output energy closed-loop control module (252), a feedback sampling adjustable module (253), a switching control logic module (254), a DC drive module (255) and an inverter drive module (256).

14. The electrosurgical generator (20) according to claim 13, **characterized in that** the feedback sampling adjustable module (253) is configured to regulate electrical quantity signals, fed back from the DC voltage and DC current sampling module (270) and from the AC voltage and AC current sampling module (280), into feedback electrical quantity signals that is able to be used by the integrated control system (251).

15. The electrosurgical generator (20) according to claim 13, **characterized in that** the integrated control system (251) is a complex control system consisting of MCU, DSP or FPGA digital control chip, which is able to output energy to the output energy closed-loop control module (252) according to a user instruction and a feedback electrical quantity, and at the same time a corresponding switching strategy is generated by the switching control logic module (254).

16. The electrosurgical generator (20) according to claim 13, **characterized in that** the DC drive module (255) controls an output voltage from the adjustable DC/DC converter (220) according to a given output from the output energy closed-loop control module (252).

17. The electrosurgical generator (20) according to claim 13, **characterized in that** the inverter drive module (256) controls inversion control of the inverter system (230) according to a given output from the output energy closed-loop control module (252).

18. The electrosurgical generator (20) according to claim 13, **characterized in that** the switching control system (257) controls the switching system (240) to perform a power circuit switching operation according to a switching strategy generated by the switching control logic module (254).

19. The electrosurgical generator (20) according to claim 11, **characterized in that** the filter system (260) consists of two groups of filters, wherein a first group acts as a high-frequency energy output filter channel, which consists of a high-frequency isolation transformer (261) and a low-pass filter (263); and a second group acts as an ultrasonic energy output filter channel, which consists of a high-frequency isolation transformer (262) and a low-pass filter (264).

20. The electrosurgical generator (20) according to claim 11, **characterized in that** the DC power supply system (210) supplies power to the adjustable DC/DC converter (220), and the adjustable DC/DC converter (220) controls outputting of a specified DC voltage to the inverter system (230) via the DC drive module (255) according to a given signal from the control system (250); an operation of the inverter system (230) is controlled by the control system (250) and driven by the inverter drive module (256), so that a DC voltage is chopped into a high-frequency AC square wave voltage Vab with different frequencies and different pulse widths as required.

21. The electrosurgical generator (20) according to claim 20, **characterized in that** a frequency of the AC square wave voltage Vab is linearly adjustable in a range of 300 kHz to 1 MHz.

22. The electrosurgical generator (20) according to claim 20, **characterized in that** the switching system (240) selects an output channel for Vab according to a given signal from the control system (250).

23. The electrosurgical generator (20) according to claim 11, **characterized in that** a main circuit of the inverter system (230) is able to adopt any form of DC/AC converter topology.

24. The electrosurgical generator (20) according to claim 23, **characterized in that** the topology comprises, but is not limited to, an H-bridge topology.

25. The electrosurgical generator (20) according to claim 11, **characterized in that** the adjustable DC/DC converter (220) adjusts a DC voltage supplied to the inverter system (230) in real time according to actual output conditions, and an output from the adjustable DC/DC converter (220) is, by default, preferentially matched with an output power from the electrosurgical generator (20), so that the inverter system (230) is always kept operating at a high DC voltage utilization rate.

26. The electrosurgical generator (20) according to claim 11, **characterized in that** the switching system (240) switches an output channel by means of a switch, wherein a switching switch is able to be an electrically controlled physical switch or an electronic switch, and a selection of the output channel is determined by a switching strategy generated by the switching control logic module (254).

27. The electrosurgical generator (20) according to claim 26, **characterized in that** the switch comprises a first switch (241) and a second switch (242), wherein during operation in a high-frequency energy output mode, the first switch (241) is closed, the second switch (242) is opened, and high-frequency energy is output to a first energy output port (291 and 292) via a high-frequency isolation transformer (261) and a low-pass filter (263); and during operation in an ultrasonic energy output mode, the first switch (241) is opened, the second switch (242) is closed, and ultrasonic energy is output to a second energy output port (293 and 294) via a high-frequency isolation transformer (262) and a low-pass filter (264).

28. The electrosurgical generator (20) according to claim 27, **characterized in that** the first energy output port (291 and 292) and the second energy output port (293 and 294) are connected in parallel, and the high-frequency energy and the ultrasonic energy are finally output through the first energy output port (291 and 292).

29. The electrosurgical generator (20) according to claim 27, **characterized in that** the first energy output port (291 and 292) and the second energy output port (293 and 294) are independent of each other, the high-frequency energy is finally output through the first energy output port (291 and 292), the ultrasonic energy is finally output through the second energy output port (293 and 294), with the both being independent of each other in terms of the output channel.

30. The electrosurgical generator (20) according to claim 20, **characterized in that** Vab in a high-frequency energy output mode is a series of AC square wave voltages with a fixed phase difference and an adjustable pulse width, with an AC square wave frequency of at least 300 kHz.

31. The electrosurgical generator (20) according to claim 20, **characterized in that** Vab in an ultrasonic energy output mode is generated by means of pulse width modulation, has a fundamental frequency, below 100 kHz, matched with a frequency required by the ultrasonic transducer (53), and has a carrier frequency at least 10 times higher than the fundamental frequency.
